Europäisches Patentamt

European Patent Office

Office européen des brevets

(19)

(11) Veröffentlichungsnummer: **0 277 282 B1**

# EUROPÄISCHE PATENTSCHRIFT

(12)

(45) Veröffentlichungstag der Patentschrift: 07.08.91

(51) Int. Cl.5: **A61F 2/44**

(21) Anmeldenummer: 87115639.4

(22) Anmeldetag: 24.10.87

(54) **Gelenkendoprothese.**

(30) Priorität: 08.01.87 CH 40/87

(43) Veröffentlichungstag der Anmeldung:
10.08.88 Patentblatt 88/32

(45) Bekanntmachung des Hinweises auf die
Patenterteilung:
07.08.91 Patentblatt 91/32

(84) Benannte Vertragsstaaten:
**AT BE DE ES FR GB IT NL SE**

(56) Entgegenhaltungen:
EP-A- 0 179 695
DE-A- 2 263 842
FR-A- 2 124 815
US-A- 3 875 595

(73) Patentinhaber: **GEBRÜDER SULZER AKTIENGE-
SELLSCHAFT**
**Zürcherstrasse 9**
**CH-8401 Winterthur(CH)**

(72) Erfinder: **Frey, Otto**
**Wallrütistrasse 56**
**CH-8400 Winterthur(CH)**
Erfinder: **Koch, Rudolf**
**Oberdorfstrasse 229**
**CH-8267 Berlingen(CH)**
Erfinder: **Planck, Heinrich M. F., Dr.-Ing.**
**Weinbergstrasse 66**
**D-7440 Nürtingen(CH)**

EP 0 277 282 B1

## Beschreibung

Die Erfindung betrifft eine Gelenkendoprothese, bestehend aus einem elastischen Prothesenkörper, dessen im implantierten Zustand dem Knochen zugewandten Stirnflächen je mit Verankerungselementen versehen sind, wobei der Prothesenkörper ein elastischer, zusammendrückbarer Hohlkörper ist dessen geschlossener Hohlraum mit einem, fliessfähigen inkompressiblen Medium gefüllt ist.

Gelenkendoprothesen der genannten Art werden vor allem als Zwischenwirbelprothesen verwendet; eine solche ist beispielsweise bekannt aus der FR-A-21 24 815. Eine Ausführungsform dieser bekannten Prothese besteht aus einem nachgiebigen, auf seinen flachen Stirnseiten aussen mit offenporigem Gewebe belegten Hohlkörper, dessen Hohlraum mit einer viskoelastischen, d.h. wenig fliessfähigen, inkompressiblen Flüssigkeit gefüllt ist; diese dient dazu, in Verbindung mit den durch Gewebe verstärkten Begrenzungen des Hohlraumes, unerwünschte Deformationen des Hohlkörpers bei Druckbelastungen zu vermeiden. Bei Belastungen nahe des Randes kommt es in dem, dem belasteten Teil des Randes "diametral gegenüberliegenden" Bereich der Prothese zu unerwünschten Zugbelastungen; diese können Lockerungen oder sogar ein Ablösen der Prothese an den Grenzflächen zwischen den Wirbelknochen und der Prothesenschale zur Folge haben.

Aufgabe der Erfindung ist es, eine Prothese der eingangs genannten Art zu schaffen, bei der die unerwünschten Zugbeanspruchungen bei einseitigen Belastungen ihres Randes möglichst vollständig vermieden sind. Diese Aufgabe wird mit der Erfindung dadurch gelöst, dass der Hohlraum durch mit Durchflussöffnungen versehene Zwischenwände in mehrere Kammern unterteilt ist, die untereinander in Fliessverbindung stehen.

Wie bei der eingangs diskutierten, bekannten Konstruktion behält der elastische kompressible Hohlkörper mit einer bei den auftretenden Belastungen inkompressiblen Flüssigkeitsfüllung ein konstantes Volumen bei; bei "dezentralen" Belastungen verlagert sich der Flüssigkeitsinhalt des Volumens jedoch in einem gedrosselten Strom aus einer "belasteten" Kammer in eine der Belastung ferne Kammer des Hohlkörpers und führt dort zu einer Dehnung von dessen elastischen Begrenzungen, wodurch Zugbelastungen zumindest weitgehend vermieden werden. Bei Bewegungen der angrenzenden Wirbel relativ zueinander wandert diese "Dehnung" durch das ganze Volumen entsprechend der momentanen lokalen Druckbelastung, wobei bei der "Verlagerung" der Flüssigkeit von einer Kammer in eine andere eine gedrosselte Strömung entsteht, die eine gedämpfte Verformung des Prothesenkörpers bewirkt.

Die Querstabilität der neuen Prothese lässt sich verbessern, wenn der Prothesenkörper aus einem torusähnlichen Ring mit einem über den Ringinnenraum strömungsverbundenen Ringhohlraum besteht, und wenn ferner der Prothesenkörper zwischen als seine Negativform profilierten Schalen gelagert ist.

Eine besonders innige Verbindung der Verankerungselemente mit den angrenzenden Wirbeln kann man erreichen, wenn diese durch Metallgitter gebildet sind, die mehrere Lagen aufweisen. Weiterhin kann eine übermässige Dehnung des Prothesenkörpers in radialer Richtung unter Druckbelastungen begrenzt werden, wenn mindestens seine radiale Begrenzung durch eine Armierung verstärkt ist.

Während die Verankerungselemente vorwiegend aus Metall, insbesondere Titan oder Titanlegierungen, gefertigt sind, besteht der Hohlkörper aus einem hochelastischen Polymer, beispielsweise einem Polyurethan. Als fliessfähige inkompressible Medien eignen sich körperverträgliche Flüssigkeiten, wie z.B. physiologische Kochsalzlösung oder - wenn eine erhöhte Viskosität erwünscht ist - Silikonöle.

Im folgenden wird die Erfindung anhand von als Ausführungsbeispiele gewählten Zwischenwirbelprothesen in Zusammenhang mit der Zeichnung näher erläutert.

Fig. 1     zeigt in einem Schnitt I-I von Fig. 2, der in Längsrichtung der Wirbelsäule verläuft, die neue Prothese, implantiert zwischen zwei Wirbeln;

Fig. 2     ist der Schnitt II-II von Fig. 1;

Fig. 3     zeigt in gleicher Darstellung wie Fig. 1 eine weitere Ausführungsform der neuen Zwischenwirbel-Prothese.

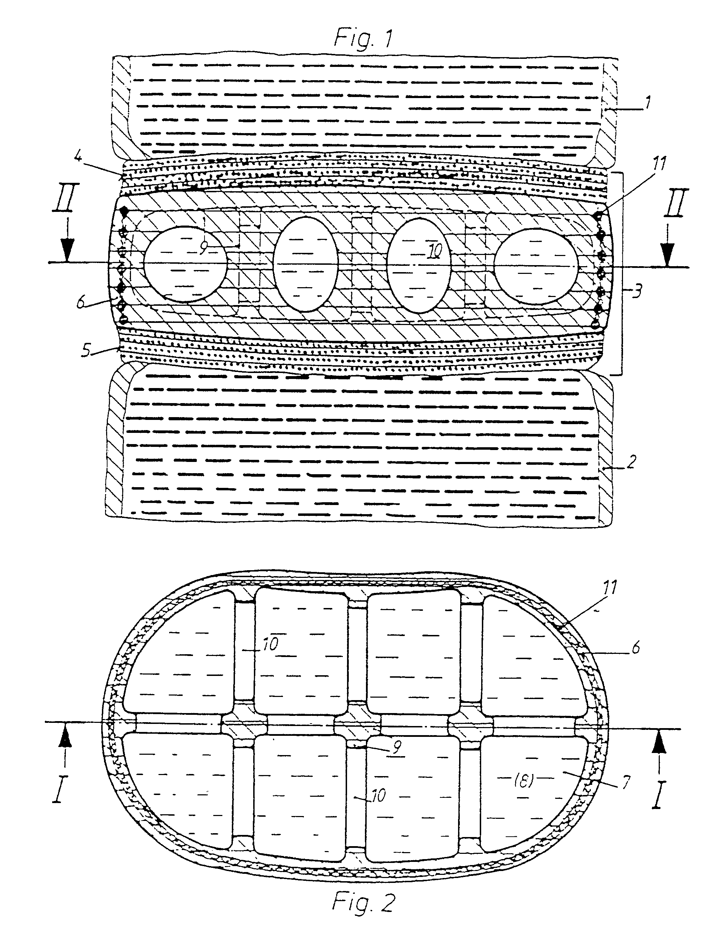

Fig. 1 zeigt schematisch zwei Wirbelkörper 1 und 2, zwischen die die neue Prothese 3 eingesetzt ist. Die Verankerungselemente sind feste Lagerschalen 4 und 5; sie bestehen aus mehreren Lagen eines metallischen Drahtnetzes, beispielsweise aus Reintitan oder einer Titanlegierung. Die aufgrund ihrer Netzstruktur porösen Oberflächen der Lagerschalen 4 und 5 sind in einem Hohl- oder Prothesenkörper 6 aus Kunststoff, beispielsweise aus Polyurethan, verankert, in dem mindestens eine Lage des Netzes in bekannter Weise (EP-A- 0 190 422) in den Kunststoff eingelassen ist. Die "offenporigen" äusseren Schichten der Lagerschalen 4 und 5 dienen dazu, die Prothese 3 durch Einwachsen von Knochengewebe mit den Wirbelkörpern 1 und 2 zu verbinden.

Der Prothesenkörper 6 ist ein kissenartiger, elastischer Hohlkörper mit einem geschlossenen Hohlraum 7. Wie erwähnt, besteht er aus einem elastischen Kunststoff. Sein Hohlraum 7 ist gefüllt mit einem fliessfähigen, inkompressiblen Medium 8, beispielsweise einer körperverträglichen Flüssigkeit, wie physiologischer Kochsalzlösung oder einem Silikonöl. Silikonöle haben dabei den Vorteil, dass durch Auswahl unter verschiedenen Oelen die Viskosität der Hohlraumfüllung in gewissen Grenzen variiert werden kann. Eine solche Variationsmöglichkeit für die Viskosität bietet auch ein Ethylenoxid/Propylenoxid-Copolymer, bei das Molekulargewicht in weiten Bereichen und die Anteile der beiden Polymere verändert werden können.

In den Hohlraum des Prothesenkörpers 6 sind Zwischenwände 9 eingezogen, die den Hohlraum 7 in mehrere - im vorliegenden Beispiel nach Fig. 1 und 2 acht - Kammern unterteilen. Die Zwischenwände 9 sind mit Durchtrittsöffnungen 10 für die Flüssigkeit 8 versehen, die bei Belastungen einen Flüssigkeitsausgleich zwischen den belasteten und den unbelasteten Kammern bewirken.

Eine unzulässige radiale Dehnung des Prothesenkörpers 6 unter Druckbelatungen wird vermieden durch eine, in seine radiale Begrenzung eingelagerte Armierung 11; diese besteht beispielsweise aus einem Gewebe, Gestrick oder Geflecht oder Gewirk oder aus einem Vlies. Als Materialien eignen sich dafür z.B. Kunst- oder Kohlenstoffasern in Mono- oder Multifilamentform mit einer geeigneten mechanischen Festigkeit und Steifigkeit.

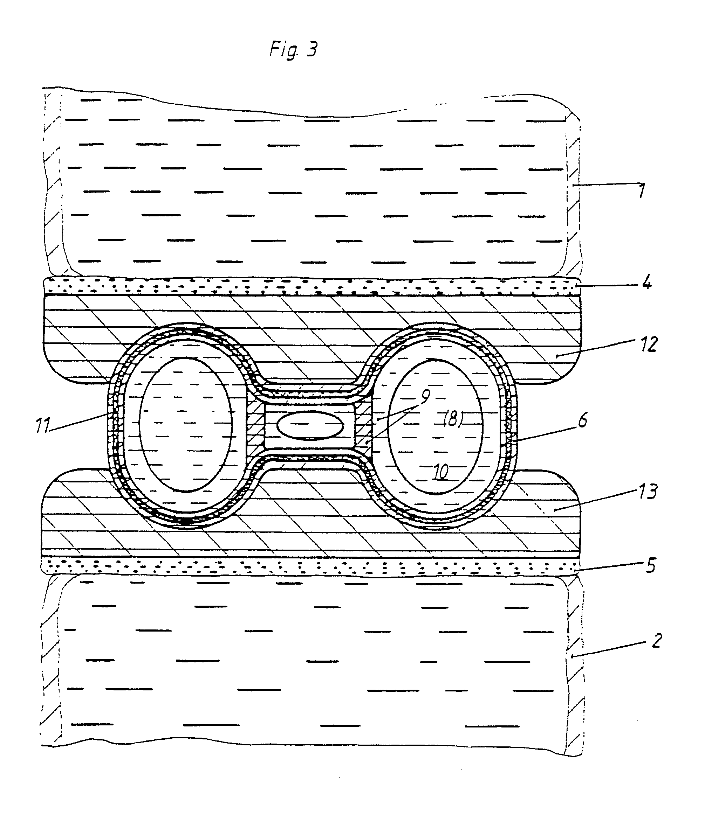

Die Ausführungsform nach Fig. 3 weist eine gegenüber dem ersten Beispiel gesteigerte Querstabilität auf, durch die ein "Schwimmen" der gezeigten Wirbel 1, 2 aufeinander in engen Grenzen gehalten wird.

Die mehrlagigen Metallgitter 4 und 5 sind prothesenseitig nicht direkt mit dem elastischen Prothesenkörper 6 verbunden, sondern an Schalen 12, 13 befestigt, die aus einem in der Implantattechnik üblichen Kunststoff bestehen; zwischen den Schalen 12, 13 ist der Prothesenkörper 6 gelagert. Dieser hat eine torusähnliche Ringform, wobei der Ringinnenraum durch einen Strömungshohlraum für das fliessfähige Medium 8 überbrückt ist, so dass dieses Medium 8 nicht nur im Ring zirkulieren kann, sondern den Ring auch diametral durchströmen kann. Zur Stabilisierung des Prothesenkörpers 6 in Querrichtung sind die Schalen 12, 13 als dessen Negativform profiliert. Wie im ersten Beispiel wird das Fliessen des Mediums 8 durch mit Oeffnungen 10 versehenen Zwischenwände 9 gedämpft und verzögert. Weiterhin ist der Prothesenkörper 6 wiederum mit einer Armierung 11 versehen, um seine "innere" Stabilität zu erhöhen.

Ausser als Zwischenwirbelprothese kann die neue Gelenkendoprothese auch als Ersatz anderer Gelenke, vor allem beispielsweise als Handgelenkprothese, dienen.

**Patentansprüche**

1. Gelenkendoprothese, bestehend aus einem elastischen Prothesenkörper (6), dessen im implantierten Zustand dem Knochen zugewandte Stirnflächen je mit Verankerungselementen (4, 5) versehen sind, wobei der Prothesenkörper (6) ein elastischer, zusammendrückbarer Hohlkörper ist, dessen geschlossener Hohlraum (7) mit einem fleissfähigen inkompressiblen Medium (8) gefüllt ist, dadurch gekennzeichnet, dass der Hohlraum (7) durch mit Durchflussöffnungen (10) versehene Zwischenwände (9) in mehrere Kammern unterteilt ist, die untereinander in Fliessverbindung stehen.

2. Prothese nach Anspruch 1, dadurch gekennzeichnet, dass die Verankerungselemente (4, 5) durch Metallgitter gebildet sind, die mehrere Lagen aufweisen.

3. Prothese nach Anspruch 1 oder 2, dadurch gekennzeichnet, dass: die Prothese eine Zwischenwirbelprothese ist und dass mindestens die radiale Begrenzung des Prothesenkörpers (6) durch eine Armierung (11) verstärkt ist.

4. Prothese nach einem der Ansprüche 1 bis 3, dadurch gekennzeichnet, dass der Prothesenkörper (6) aus einem torusähnlichen Ring mit einem über den Ringinnenraum strömungsverbundenen Ringhohlraum besteht, und dass ferner der Prothesenkörper (6) zwischen als seine Negativform profilierten Schalen gelagert ist.

**Claims**

1. A joint endoprosthesis, consisting of an elastic prosthesis body (6), whose end surfaces facing the bone in the implanted condition are each provided with anchoring elements (4, 5), the prosthesis body (6) being an elastic compressible hollow body, the closed cavity (7) of which is filled with a flowable incompressible medium (8), characterised in that the cavity (7) is divided, by partitions (9) provided with flow apertures (10), into a plurality of chambers which are in flow communication with one another.

2. A prosthesis according to claim 1, characterised in that the anchoring elements (4, 5) are formed by metal grids comprising a plurality of layers.

3. A prosthesis according to claim 1 or 2, characterised in that the prosthesis is an inter-vertebral prosthesis and in that at least the radial boundary of the prosthesis body (6) is reinforced by a reinforcement (11).

4. A prosthesis according to any one of claims 1 to 3, characterised in that the prosthesis body (6) consists of a ring after the style of a torus having an annular cavity in flow-communication via the interior of the ring, and in that the prosthesis body (6) is also mounted between shells (6) profiled as its negative form.

**Revendications**

1. Endoprothèse pour articulations constituée d'un corps de prothèse (6) élastique dont les faces frontales tournées vers l'os, à l'état implanté, sont pourvues chacune d'éléments d'ancrage (4, 5), le corps de prothèse (6) étant un corps creux élastique, compressible, dont la cavité (7) fermée est remplie d'un fluide (8) incompressible, caractérisée en ce que la cavité (7) est subdivisée, par des cloisons (9) pourvues d'ouvertures de passage (10), en plusieurs chambres qui communiquent entre elles.

2. Prothèse selon la revendication 1, caractérisée en ce que les éléments d'ancrage (4, 5) sont formés par des grilles métalliques qui comportent plusieurs couches.

3. Prothèse selon la revendication 1 ou 2, caractérisée en ce qu'elle est une prothèse intervertébrale et en ce que la délimitation radiale au moins du corps de prothèse (6) est renforcée par une armature (11).

4. Prothèse selon l'une des revendications 1 à 3, caractérisée en ce que le corps de prothèse (6) est constitué par un anneau torique avec une cavité annulaire reliée, en ce qui concerne l'écoulement, par l'espace intérieur annulaire et en ce qu'en outre le corps de prothèse (6) est monté entre des coques, profilées suivant sa forme en négatif.

## Fig. 1

## Fig. 2

Fig. 3